# EUROPEAN PATENT APPLICATION

(11) **EP 4 497 435 A1**
(43) Date of publication of application: **29.01.2025**
(21) Application number: 23774937.9
(22) Date of filing: 22.03.2023
(51) Int. Cl.: A61K 31/132, A61P 21/00

(54) **SKELETAL MUSCLE ENHANCING AGENT**

(30) Priority: 22.03.2022 JP 2022045904
(71) Applicant: Sato Pharmaceutical Co., Ltd., Tokyo 107-0051 (JP); Keio University, Tokyo, 108-8345 (JP)
(72) Inventor: OKUBO Kazumasa, Tokyo 140-0011 (JP); ITOH Yoshiki, Tokyo 140-0011 (JP); NAKAMURA Masaya, Tokyo 160-8582 (JP); TSUJI Osahiko, Tokyo 160-8582 (JP); HORIUCHI Keisuke, Tokorozawa-shi, Saitama 359-8513 (JP)
(74) Representative: Gulde & Partner
(86) International application number: PCT/JP2023/011182
(87) International publication number: WO 2023/182342

(57) **Abstract**

The present invention is to provide a means for enhancing skeletal muscle. According to the present invention, the skeletal muscle is enhanced by using spermidine or a pharmaceutically acceptable salt thereof is administered intramuscularly as an active ingredient.

## Description

### TECHNICAL FIELD

The present invention relates to a skeletal muscle enhancer comprising spermidine or a pharmaceutically acceptable salt thereof, which is administered intramuscularly.

### BACKGROUND ART

The homeostasis of skeletal muscle is controlled by a synthesis system and a degradation system (Non Patent Literature 1). If the maintenance of homeostasis fails and the degradation system becomes dominant, a decrease in skeletal muscle mass (hereinafter, also referred to as "muscle atrophy") occurs.

As causes of muscle atrophy, aging, immobility, neurodegenerative diseases, cachexia (undernutrition caused by cancer or the like), and the like are known.

The muscle atrophy that frequently occurs in the elderly due to aging is also called sarcopenia (Non Patent Literature 2). If sarcopenia is developed and muscle atrophy progresses in the whole body, activities of daily living decrease and care and support are required, and thus sarcopenia is a major problem in an aging society. Further, sarcopenia is considered as one cause of chronic low back pain (Non Patent Literature 11).

Muscle atrophy caused by immobility is also called disuse muscle atrophy. Disuse muscle atrophy occurs due to plaster casting for treatment of a bone fracture caused by injury or osteoporosis, prolonged bed rest (bedridden) due to aging, or the like.

For muscle atrophy, exercise therapy for increasing the skeletal muscle mass is effective, but development of a therapy also applicable to a person who is difficult to exercise (drug therapy or the like) is desired.

As a mechanism of muscle atrophy, enhancement of activity of muscle-specific ubiquitin proteasome systems and involvement of a negative regulator of muscle growth are known (Non Patent Literature 1).

Tirm63 (MuRF1) is a representative muscle-specific ubiquitin ligase involved in muscle atrophy, and it has been reported that muscle atrophy is suppressed by depletion of its expression (Non Patent Literature 3).

Myostatin (Mstn) is a negative regulator of muscle growth, and it has been reported that if its expression is suppressed, the muscle mass remarkably increases (Non Patent Literature 4).

There is skeletal muscle damage (hereinafter, also referred to as "muscle damage") as a decrease in the skeletal muscle mass occurs due to causes different from the disruption of homeostasis.

Muscle damage is clinically classified into muscle strain (pulled muscle), high energy trauma, damage caused by surgical operation, and the like. Muscle damage is known to cause various complications (dysfunction, muscle atrophy, local pain, and the like) (Non Patent Literature 9).

Among muscle damages, muscle strain caused by blunt external force (bruise) is the most frequent sports injury (Non Patent Literature 10). Although there are no accurate statistics, it is considered that the number of patients in Japan is about several tens of thousands per year.

As a first aid to muscle damage, RICE treatment (Rest, Icing, Compression, Elevation) has been recommended so far. After the first aid treatment, symptomatic treatment for pain, rehabilitation, and the like have been conducted.

Skeletal muscle tissue has a mechanism for regeneration from the damage. It is known that muscle satellite cells are essential for muscle regeneration. The muscle satellite cells that reside in the vicinity of the muscle fiber basement membrane, exist in a quiescent state under normal conditions, but when muscle damage is caused, the cells are activated and differentiated into myoblasts and form muscle fibers via cell fusion. When the muscle regeneration is completed, the remaining muscle satellite cells enter the quiescent state again (Non Patent Literatures 5 and 6).

As the factor that promotes muscle regeneration, a hepatocyte growth factor (HGF) (Non Patent Literature 7) and an insulin-like growth factor 1 (IGF-1) (Non Patent Literature 8) are known. Further, it has been reported that muscle hypertrophy can be induced by suppressing a factor that inhibits muscle regeneration (Patent Literature 1).

However, it takes a long time to regenerate the muscle by the above mechanism. As a result, conventional treatments that do not take particular measures to promote muscle regeneration cause muscle weakness and delay the return of muscle damaged patients to daily life and sports activities.

Further, if it takes time to regenerate the muscle, part of muscle tissue may be replaced with the scar tissue derived from collagen that has remained in the muscle tissue for a long period of time. Since the scar tissue reduces the strength of plastic muscles, the risk of recurrence of muscle damage is high (Non Patent Literature 9).

It is known that muscle regeneration is promoted when the homeostasis of skeletal muscle becomes dominant in the synthesis system, leading to the formation of muscle fibers (Non Patent Literatures 5 and 6).

### Citation List

### Patent Literature

Patent Literature 1: WO 2013/039244 A

### Non Patent Literature

Non Patent Literature 1: Physiology, vol. 23, no. 3, pp. 160-170, 2008
Non Patent Literature 2: Sarcopenia Muscle, vol. 10, no. 5, pp. 956-961, 2019
Non Patent Literature 3: Science (80-.)., vol. 294, no. 5547, pp. 1704-1708, 2001
Non Patent Literature 4: Cell. Mol. Life Sci., vol. 71, no. 22, pp. 4361-4371, 2014
Non Patent Literature 5: Am. J. Sports Med., vol. 33, no. 5, pp. 745-64, May 2005
Non Patent Literature 6: J. Bone Joint Surg. Am., vol. 84-A, no. 5, pp. 822-32, May 2002
Non Patent Literature 7: Dev. Biol., vol. 194, no. 1, pp. 114-128, 1998
Non Patent Literature 8: J. Cell. Physiol., vol. 138, no. 2, pp. 311-5, Feb. 1989
Non Patent Literature 9: J. Appl. Physiol., vol. 95, no. 2, pp. 771-780, 2003
Non Patent Literature 10: Am. J. Sports Med., vol. 27, no. 1, pp. 2-9, 1999
Non Patent Literature 11: Spine & Spinal Cord., vol. 30, no. 5, pp. 573-578, 2017

### SUMMARY OF INVENTION

### Technical Problem

In the conventional treatments for muscle atrophy and muscle damage, there are problems such as reduction in motor function and healthy life expectancy of patients, or the long-term suspension of activities of athletes due to a limited application range, requirement of a long recovery time, ease of occurrence of muscle weakness, and the like. Further, although the molecular mechanism of muscle atrophy and muscle regeneration has been widely studied, an effective drug has not been developed yet. Therefore, the development of a drug that enhances skeletal muscle has been strongly desired.

### Solution to Problem

The present inventors performed intensive studies to solve the problems, and as a result, found that when spermidine is administered intramuscularly to a subject suffering from muscle atrophy or muscle damage, suppresses the degradation system or promotes the synthesis system in skeletal muscle homeostasis, resulting in an increase in skeletal muscle weight (that is, skeletal muscle is enhanced), and completed the present invention. That is, the present invention relates to the following [1] to [10].
[1] A skeletal muscle enhancer comprising spermidine or a pharmaceutically acceptable salt thereof, which is administered intramuscularly.
[2] The skeletal muscle enhancer described in the above [1], wherein the skeletal muscle enhancer is administered to a subject having skeletal muscle atrophy.
[3] The skeletal muscle enhancer described in the above [1] or [2], wherein the skeletal muscle atrophy is sarcopenia or disuse muscle atrophy.
[4] The skeletal muscle enhancer described in the above [1], wherein the skeletal muscle enhancer is administered to a subject having skeletal muscle damage.
[5] The skeletal muscle enhancer described in the above [4], wherein the skeletal muscle damage is a muscle strain.
[6] The skeletal muscle enhancer described in the above [4], wherein the skeletal muscle damage is due to myogenic disease.
[7] The skeletal muscle enhancer described in any one of the above [1] to [6], wherein the skeletal muscle is enhanced by suppression of degradation or promotion of synthesis of skeletal muscle.
[8] A therapeutic agent for skeletal muscle atrophy comprising spermidine or a pharmaceutically acceptable salt thereof, which is administered intramuscularly.
[9] A therapeutic agent for skeletal muscle damage comprising spermidine or a pharmaceutically acceptable salt thereof, which is administered intramuscularly.
[10] A skeletal muscle homeostasis restoring agent comprising spermidine or a pharmaceutically acceptable salt thereof, which is administered intramuscularly.

### Effects of Invention

As shown in Examples to be described later, the skeletal muscle enhancer according to the present invention can enhance skeletal muscle.

### BRIEF DESCRIPTION OF DRAWINGS

Fig. 1 shows the effect on skeletal muscle weight by intramuscular (i.m.) administration of spermidine trihydrochloride (SPD) to the muscle atrophy model.
Fig. 2 shows the effect on the muscle fiber area by intramuscular (i.m.) administration of SPD to the muscle atrophy model.
Fig. 3 shows the effect on Trim63 gene expression by SPD administration to the muscle atrophy model.
Fig. 4 shows the effect on Mstn gene expression by SPD administration to the muscle atrophy model.
Fig. 5 shows the effect on skeletal muscle weight by SPD administration to the muscle damage model.
Fig. 6 shows the effect on the regenerated muscle area by intramuscular (i.m.) administration of SPD to the muscle damage model.
Fig. 7 shows the effect on the regenerated muscle area by intraperitoneal (i.p.) administration of SPD to the muscle damage model.
Fig. 8 shows the effect on the regenerated muscle area by oral administration of SPD to the muscle damage model.

### DESCRIPTION OF EMBODIMENTS

### [Active ingredient]

The skeletal muscle enhancer according to the present invention contains spermidine or a pharmaceutically acceptable salt thereof as an active ingredient.

Spermidine (H₂N(CH₂)₄NH(CH₂)₃NH₂) is a type of polyamine presents in many organisms.

As the pharmaceutically acceptable salt of spermidine (hereinafter, also referred to as the "salt thereof"), for example, a hydrochloride, a phosphate, etc. are mentioned.

The salt of spermidine include solvate with a pharmaceutically acceptable solvent such as water or ethanol.

Spermidine and a salt thereof are known substances, and are readily available on the market, or easily produced by known means.

### [Skeletal muscle enhancer]

The term "skeletal muscle enhancement" used herein means the increase of the skeletal muscle mass reduced by muscle atrophy, muscle damage, or the like. Although the present invention is not limited by a specific theory, it is considered that the increase in the skeletal muscle mass is achieved by suppression of the degradation system or promotion of the synthesis system in the homeostasis of skeletal muscle, whereby the synthesis system becomes dominant (muscle regeneration is promoted), and the number of single muscle fibers constituting the skeletal muscle is increased and/or the single muscle fibers themselves are enlarged, or the like.

The term "muscle atrophy" used herein means the decrease in the skeletal muscle mass caused by the predominance of the degradation system among the synthesis system and the degradation system that maintain homeostasis of skeletal muscle.

As the cause of muscle atrophy, aging, immobility, neurodegenerative diseases (such as amyotrophic lateral sclerosis and spinal muscular atrophy), and cachexia, are mentioned.

The term "muscle damage" used herein means the decrease in the skeletal muscle mass caused by a cause different from the disruption of homeostasis (such as external force or necrosis of muscle fibers due to myogenic disease).

As the muscle damage due to an external force, for example, muscle strain (caused by external force (for example, a bruise or the like)), pulled muscle (caused by internal force such as a rapid contraction of muscle), and cervical sprain (so-called whiplash), are mentioned.

As the myogenic disease that causes muscle damage, for example, muscular dystrophy and distal myopathy, etc. are mentioned.

In this regard, muscle damage due to an external force and muscle damage due to myogenic disease are common to each other in that muscle regeneration compensating for necrosis of muscle fibers (muscle damage) occurs.

Skeletal muscle enhancers used in drug therapy are suitable for treatment of subjects having difficulty in exercise therapy, particularly, subjects having muscle atrophy due to aging (sarcopenia), muscle atrophy due to disuse muscle atrophy, muscle damage due to external force (in particular, muscle strain (pulled muscle)), or muscle damage due to myogenic disease.

The concentration of spermidine or a salt thereof in a skeletal muscle enhancer can be appropriately set depending on the degree of muscle atrophy, muscle damage, and the like.

The skeletal muscle enhancers can be applied to an animal having skeletal muscle without any limitation. The application target is preferably a mammal (a human, or a non-human mammal (for example, a horse or a cow)), and more preferably a human. Further, there are no restrictions on the sex and age of the application target.

### [Pharmaceutical formulation for intramuscular administration]

The skeletal muscle enhancer is administered intramuscularly into skeletal muscle. Intramuscular administration exerts a higher effect than other administration routes.

As the formulation for intramuscular administration, for example, a sterilized pharmaceutical formulation in a liquid state such as a solution or a suspension, specifically, or an injection can be mentioned.

The formulation can be conducted by using a common formulation technique.

The pharmaceutical formulation for intramuscular administration may contain a pharmaceutically acceptable solvent or diluent in addition to an active ingredient.

As the "pharmaceutically acceptable solvent or diluent", for example, distilled water for injection, a lidocaine hydrochloride aqueous solution (for intramuscular injection), a saline solution, an aqueous solution of glucose, ethanol, polyethylene glycol, propylene glycol, a liquid for intravenous injection (for example, an aqueous solution of citric acid, sodium citrate, or the like), an electrolyte solution (for intravenous drip infusion or intravenous injection), and a mixed solution thereof, etc. are mentioned.

The injection may be prepared in the form of predissolved active ingredient, and further may be prepared in the form that is dissolved at the time of use as a powder of the active ingredient as it is or a powder of the active ingredient added with an appropriate carrier. The injection can contain, for example, 0.005 to 25% by mass of an active ingredient based on the total mass of the pharmaceutical formulation.

### [Therapeutic agent for atrophy or damage of skeletal muscle]

Spermidine and salts thereof can treat muscle atrophy and muscle damage by enhancing skeletal muscle. Accordingly, the skeletal muscle enhancer according to the present invention can be grasped also as a therapeutic agent for atrophy or damage of skeletal muscle.

The description about the active ingredient and formulation of the skeletal muscle enhancer is applied to the therapeutic agent.

### [Skeletal muscle homeostasis restoring agent]

Spermidine and salts thereof can restore homeostasis inclined to the degradation side in muscle atrophy by suppressing the degradation system or promoting the synthesis system. Accordingly, the skeletal muscle enhancer according to the present invention can be grasped also as a skeletal muscle homeostasis restoring agent.

The description about the active ingredient and formulation of the skeletal muscle enhancer is applied to the restoring agent.

### EXAMPLES

Next, the effects of the present invention will be specifically described by way of Examples, however, the present invention is not limited to these Examples.

### [Experimental method]

### 1. Evaluation compound

Spermidine trihydrochloride (Nacalai Tesque, Inc.)

### 2. Test animals

Seven-week old C57BL/6 Male mice (CLEA Japan, Inc.) were purchased, and used for experiment at the age of 8 weeks.

### 3. Muscle-atrophy model

A model animal to which muscle atrophy had been caused by a method of fixing a hindlimb (J Appl Physiol, vol. 106, pp. 2049-2059, 2009) was used.

Under the anesthesia with isoflurane, the left hindlimb of the mouse in a bent state was fixed with a surgical stapler (Kaiser Plus. KEISEI MEDICAL INDUSTRIAL CO., LTD.). The right hindlimb of the same mouse was assigned to the sham-treatment side, and no fixation treatment was performed. After 3 or 7 days of the start of fixation, the tibialis anterior muscle (one of skeletal muscles) was collected by dissection. The sample collected after 3 days was supplied to the expression analysis of the muscle atrophy-related genes, and the sample collected after 7 days was supplied to the measurement of a muscle weight and the preparation of a muscle tissue section.

### 4. Expression analysis of muscle atrophy-related genes

The tibialis anterior muscle was homogenized with Sepasol-RNA I Super G (NACALAI TESQUE, INC.), 1-bromo-3-chloropropane was added thereto, the mixture was stirred, and further centrifuged, then RNA was extracted from the supernatant. The extracted RNA was recovered with 2-propanol and 75% ethanol, and then subjected to the reverse transcription reaction (using ReverTra Ace qPCR RT Master Mix (Toyobo Co., Ltd.) according to the attached protocol) to synthesize cDNA. The synthesized cDNA was subjected to the real-time PCR method (using Applied Biosystems 7500 Real-Time PCR System (Thermo Fisher Scientific)) to measure mRNA expression of three genes, Trim63 (MuRF1), myostatin (Mstn), and Gapdh. Primer sequences used are as follows.

| | | |
|---|---|---|
| Trim63 | Sense strand | CCAGGCTGCGAATCCCTAC (SEQ ID NO: 1) |
| | Antisense strand | ATTTTCTCGTCTTCGTGTTCCTT (SEQ ID NO: 2) |
| Mstn | Sense strand | AGTGGATCTAAATGAGGGCAGT (SEQ ID NO: 3) |
| | Antisense strand | GTTTCCAGGCGCAGCTTAC (SEQ ID NO: 4) |
| Gapdh | Sense strand | TCAACAGCAACTCCCACTCTTCCA (SEQ ID NO: 5) |
| | Antisense strand | ACCCTGTTGCTGTAGCCGTATTCA (SEQ ID NO: 6) |

Tirm63 (MuRF1) has been reported to suppress muscle atrophy by depletion of its expression (Non Patent Literature 3), and is a gene that acts on enhancement of the degradation system in the homeostasis of skeletal muscle.

Myostatin (Mstn) has been reported to be a negative regulator of muscle growth (Non Patent Literature 4), and is a gene that acts on suppression of the synthesis system (in other words, enhancement of the degradation system) in the homeostasis of skeletal muscle.

Gapdh was used as a control for normalizing the expression levels of muscle atrophy-related genes.

The expression levels of mRNA of Trim63 and Mstn (mTrim63 and mMstn) were calculated as a ratio to the expression level of mRNA of Gapdh (mGapdh).

The evaluation was performed based on the proportion (%) of the expression level in the other administration group when the expression level in the fixed left hind paw of the Vehicle administration group (details will be described later) was set to 100%.

### 5. Muscle-damage model

A model animal to which muscle damage had been caused by administion of snake venom cardiotoxin (CTX) was used. The model animal has been widely used in studies on regeneration from muscle damage.

Under the anesthesia with isoflurane, 50 µL of 10 µM CTX was administered to the tibialis anterior muscle of the right hindlimb of the mouse. Fifty µL of a phosphate buffer (PBS) was administered as a Vehicle to the tibialis anterior muscle of left hindlimb of the same mouse. After 7 days of the administration of CTX, the tibialis anterior muscle was collected by dissection, and supplied to the measurement of muscle weight and preparation of a muscle tissue section.

### 6. Measurement of muscle weight

Body weight of the mouse was measured before collecting the tibialis anterior muscle. Furthermore, the weight of the collected tibialis anterior muscle was measured. From these measured values, the ratio (%) of the skeletal muscle weight per body weight of the mouse (weight of tibialis anterior muscle/body weight of mouse) was calculated.

### 7. Preparation of muscle tissue section

Immediately after the collection of the tibialis anterior muscle, the tibialis anterior muscle was immersed in isopentane cooled with liquid nitrogen and was rapidly frozen. The frozen muscle tissue was cut into slices each having a thickness of 10 µm by using a cryostat (Leica Biosystems), and the slice was attached onto an antistripping coated slide glass (Matsunami Glass Ind., Ltd.).

### 8. Immunofluorescence staining of muscle tissue section

A muscle tissue section was sufficiently air dried for 30 minutes under room temprature. After that, the muscle tissue sections was fixed by immersing it in acetone cooled to -30°C and treating at -30°C for 20 minutes. The fixed sections was air dried once and washed with PBS. Then the section was blocked by dropwisely adding a blocking reagent (Blocking One. NACALAI TESQUE, INC.) to the section and subjected to the blocking treatment for 1 hour. Next, a primary antibody (Anti-laminin-2 (α-2 Chain) antibody Rat monoclonal (Sigma-Aldrich)) obtained by being diluted 500 times with a blocking reagent was added dropwise, and the reaction was conducted at overnight at 4°C. Since laminin to which a primary antibody binds is a protein expressed in the basement membranes of all muscle cells, the laminin was used in this experiment as an index in order to measure the area of individual muscle cells in a section. The muscle tissue section after the reaction with the primary antibody was washed with PBS, and then was reacted for 1 hour with a secondary antibody (CF 488A Goat Anti-Rat IgG (H+L) (Biotium)) obtained by being diluted 500 times with the blocking reagent. The secondary antibody that is an anti-rat antibody conjugated with a fluorescent dye binds to the primary antibody, and stains the laminin. The muscle tissue sections after the reaction with the secondary antibody was washed with PBS, and sealed by using "VECTASHIELD Hard·Set with DAPI" (Vector), and then the fluorescence observation was performed with an inverted microscope FSX100 (Olympus).

### 9. Measurement of muscle fiber cross-sectional area

The muscle fiber cross-sectional area was measured basis of the image data taken from the fluorescence observation. After the image data was taken into image analysis software ImageJ (NIH), the cross-sectional area of the individual cells were measured on the basis of the cell membrane stained with laminin. For the area measurement, "Analyze Particles" that is an add-in analysis program on ImageJ was used. The measurement results were shown as a distribution chart (histogram) of the areas and number of single-muscle fibers, and as an average value of the cross-sectional areas of all the single muscle fibers (mean muscle fiber area).

In the muscle damage model (Example 3), the regenerated muscle was used as a measurement target. A central nucleus fiber (single muscle fiber having a central nucleus) having a nucleus dyed with DAPI contained in the mounting medium at the center of the cell was determined to be a regenerated muscle, and was distinguished from a muscle (not having the central nucleus) existing before the regeneration occurred.

In the muscle atrophy models (Examples 1 and 2), no distinction based on the central nucleus was made, and the whole muscle was used as a measurement target.

### [Example 1]

Spermidine trihydrochloride (SPD) was administered intramuscularly to muscle atrophy model animals, and the variations of skeletal muscle weight and skeletal muscle fiber cross-sectional area were evaluated.

In an intramuscular administration group, a preparation made by dissolving SPD in a PBS (SPD concentration: 196 mM) was used. This preparation (volume: 10 µL) was injected into the tibialis anterior muscle of legs of a model once a day from the day before the left hindlimb fixation to the day before dissection.

In a control, PBS (Vehicle) was injected once a day from the day before the left hindlimb fixation to the day before dissection.

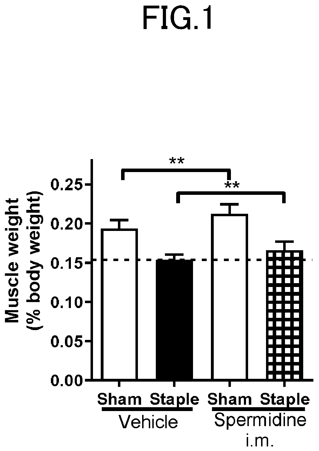

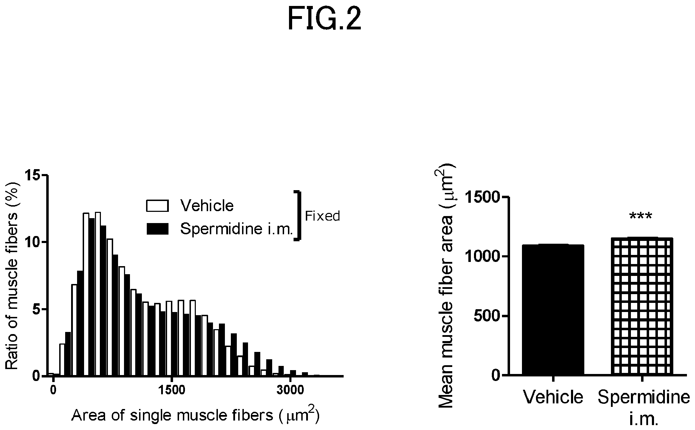

After 7 days of the start of fixation, the tibialis anterior muscle was collected, and the muscle weight and the number and area of single-muscle fibers were measured by the above method. The results are shown in Fig. 1 (muscle weight) and Fig. 2 (single-muscle fiber area).

Regarding the effect of hindlimb fixation on the muscle weight, a significant weight loss was observed in the Vehicle administration group (control) (Fig. 1).

Regarding the effect of SPD administration on the muscle weight, a significant increase in the muscle weight was observed in the intramuscular administration group as compared with the Vehicle administration group (control) in both the right hindlimb (sham treatment: Sham) and the left hindlimb (fixed: Staple) (Fig. 1). Further, the intramuscular administration group showed a significant suppression of the muscle weight loss due to hindlimb fixation (Fig. 1, ** P < 0.01).

When the evaluation was made on the basis of the single-muscle fiber area, the histogram of the SPD intramuscular administration group was shifted to the right side (in a direction in which the area becomes larger) as compared with histogram of the Vehicle administration group, and a significant increase in the mean muscle fiber area was observed (Fig. 2, *** P < 0.001).

These results indicate that, by intramuscular administration of SPD, in subjects having normal skeletal muscle and subjects having disuse muscle atrophy, the degradation system was suppressed or the synthesis system was promoted (skeletal muscle is enhanced) in the homeostasis of skeletal muscle.

### [Example 2]

Spermidine trihydrochloride (SPD) was administered to muscle atrophy model animals by three administration routes, and the expression variations of muscle atrophy-related genes Trim63 and Mstn were measured.

In an intramuscular administration group, a preparation made by dissolving SPD in a PBS (SPD concentration: 196 mM) was used. This preparation (volume: 10 µL) was injected into the tibialis anterior muscle of both legs of a model once a day from the day before the left hindlimb fixation to the day before the dissection.

In an intraperitoneal administration group, a preparation made by dissolving SPD in a PBS (SPD concentration: 19.6 mM) was used. This preparation (volume: 10 mL/kg) was injected intraperitoneally into a model once a day from the day before the left hindlimb fixation to the day before the dissection.

In an oral administration group, a preparation made by dissolving SPD in water (SPD concentration: 30 mM) was used. This preparation was administered orally by free drinking from the day before the left hindlimb fixation to immediately before dissection. The mean water intake during the oral administration period was 2.26 mL/day per mouse.

In a control of the intramuscular administration and intraperitoneal administration groups, PBS (Vehicle) was administered once a day from the day before the left hindlimb fixation to the day before the dissection. Also, water was used as a control in the oral administration group.

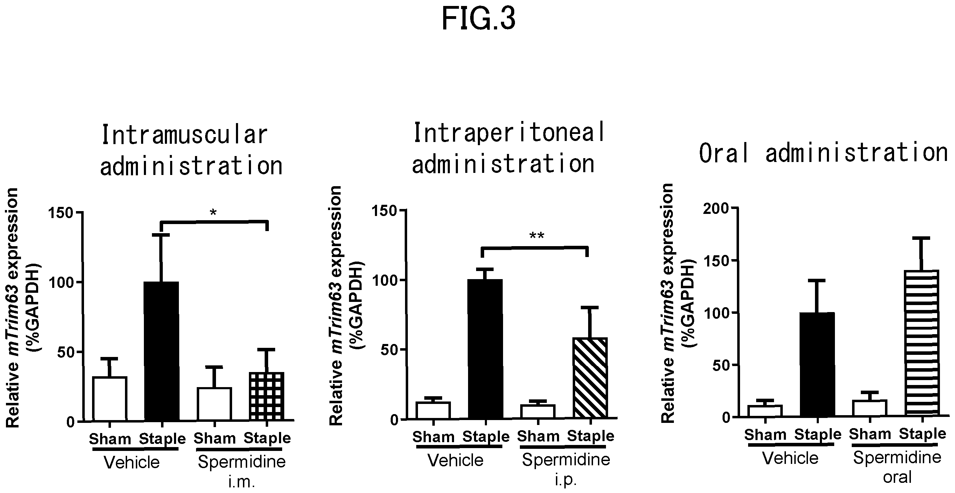

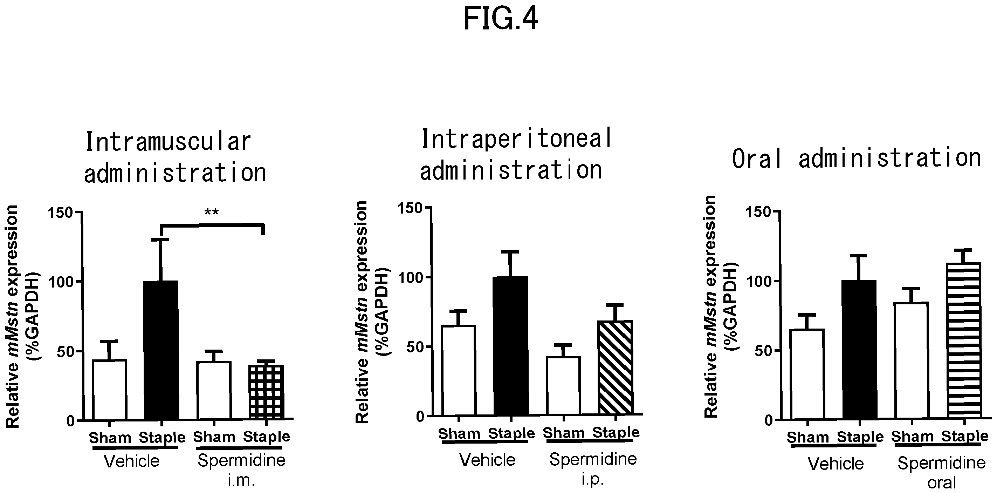

The mRNA expression of Trim63 and Mstn in the tibialis anterior muscle of the left hindlimb (fixed: Staple) and the right hindlimb (sham treatment: Sham) collected after 3 days of the start of fixation was measured. The results are shown in Figs. 3 and 4.

Regarding the effect of hindlimb fixation on Trim63 expression, a remarkable increase in expression was observed in the Vehicle administration group (control) regardless of the administration route (Fig. 3).

Regarding the effect of SPD administration on Trim63 expression, the intramuscular administration group (Staple, Spermidine) showed the significant expression suppression of 65.6% reduction as compared with the Vehicle administration group (Staple, Vehicle) (Fig. 3, * P < 0.05). On the other hand, in the intraperitoneal administration group (Staple, Spermidine), the expression suppression was reduced only by 42.3% as compared with the Vehicle administration group (Staple, Vehicle), and the oral administration group showed no expression suppression (Fig. 3, ** P < 0.01).

Regarding the effect of hindlimb fixation on Mstn expression, a remarkable increase in expression was observed in the Vehicle administration group (control) regardless of the administration route.

Regarding the effect of SPD administration on Mstn expression, the intramuscular administration group (Staple, Spermidine) showed the significant expression suppression of 61.1% reduction as compared with the Vehicle administration group (Staple, Vehicle) (Fig. 4, ** P < 0.01). On the other hand, the intraperitoneal administration group and the oral administration group did not show significant expression suppression (Fig. 4).

Based on these results, it is understood that the intramuscular administration of SPD is possible to enhance the skeletal muscle by suppressing the expression of genes that promote the degradation system in the homeostasis of skeletal muscle, thereby making the synthesis system dominant.

### [Example 3]

Spermidine trihydrochloride (SPD) was administered to a muscle damage model by three administration routes, and variations of skeletal muscle weight and regenerated skeletal muscle fiber cross-sectional area were evaluated.

In an intramuscular administration group, a preparation made by dissolving SPD in a PBS (SPD concentration: 196 mM) was used. This preparation (volume: 10 µL) was injected into the tibialis anterior muscle of both legs of a model once a day from the day before CTX administration to the day before the dissection.

In an intraperitoneal administration group, a preparation made by dissolving SPD in a PBS (SPD concentration: 19.6 mM) was used. This preparation (volume: 10 mL/kg) was injected intraperitoneally into a model once a day from the day before CTX administration to the day before the dissection.

In an oral administration group, a preparation made by dissolving SPD in water (SPD concentration: 30 mM) was used. This preparation was administered orally by free drinking from the day before CTX administration to immediately before dissection. The mean water intake during the oral administration period was 2.32 mL/day per mouse.

In a control of the intramuscular administration and intraperitoneal administration groups, PBS (Vehicle) was administered once a day from the day before CTX administration to the day before the dissection. Also, water was used as a control in the oral administration group.

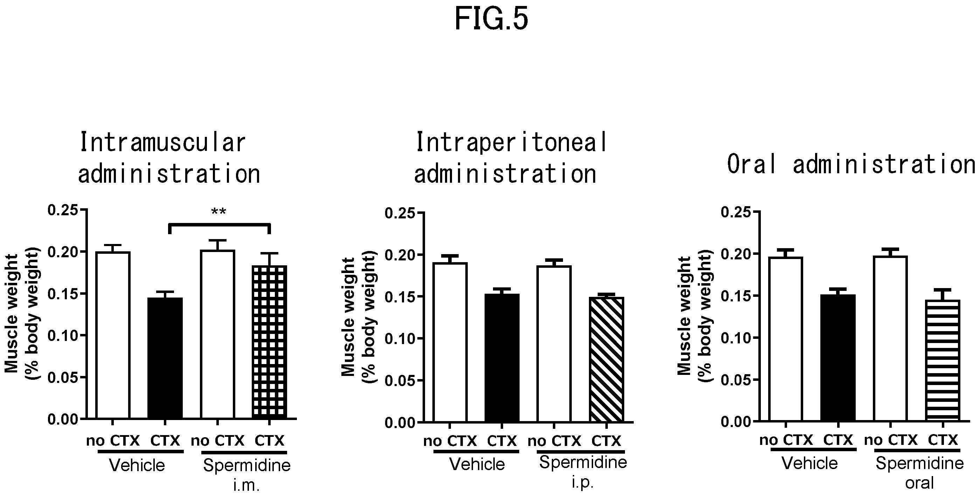

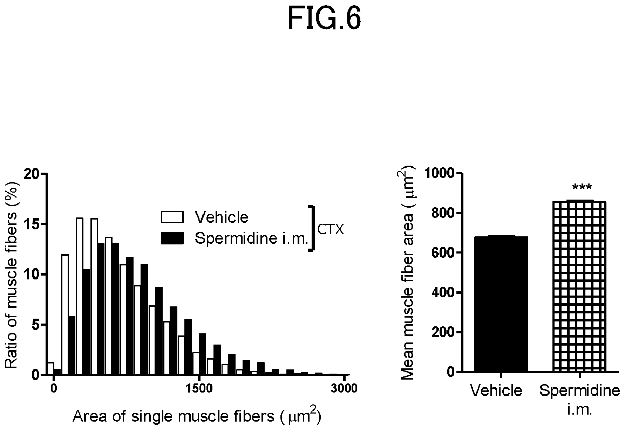

After 7 days of the administration of CTX, the tibialis anterior muscle was collected, and the muscle weight and the number and area of regenerated single-muscle fibers were measured by the above method. The results are shown in Fig. 5 (muscle weight) and Fig. 6 to 8 (regenerated single-muscle fiber area).

Regarding the effect of CTX administration on the muscle weight, in the Vehicle administration group (control), a remarkable weight loss was observed regardless of the administration route (Fig. 5, ** P < 0.01).

Regarding the effect of SPD administration on the muscle weight, a remarkable suppression of the muscle weight loss was observed in the intramuscular administration group, but a significant suppression was not observed in the intraperitoneal administration group and the oral administration group (Fig. 5).

When muscle regeneration was evaluated on the basis of the single-muscle fiber area, in the intramuscular administration group, the histogram of the SPD administration group was shifted to the right side (in a direction in which the area become larger) as compared with histogram of the Vehicle administration group, and a significant increase in the mean muscle fiber area was observed (Fig. 6, *** P < 0.001).

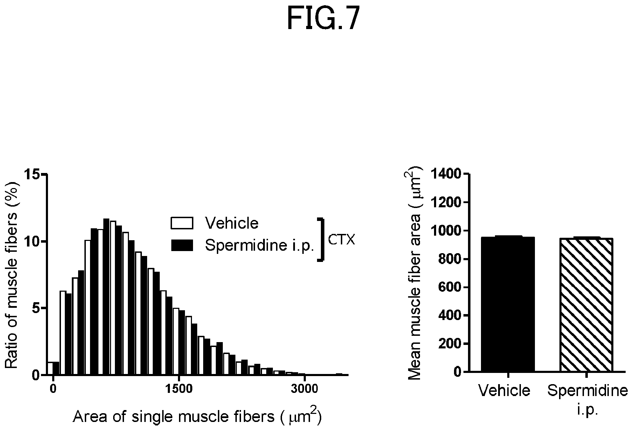

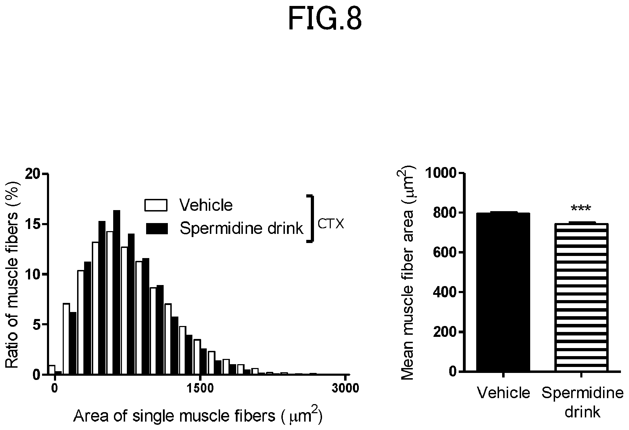

On the other hand, in the intraperitoneal administration group and the oral administration group, no shift due to SPD administration was observed in the histogram, and no significant increase in the mean muscle fiber area was observed (Figs. 7 and 8).

These results indicate that intramuscular administration of SPD promoted regeneration of skeletal muscle (enhanced skeletal muscle) in subjects having muscle damage.

### Industrial Applicability

By intramuscularly administering the skeletal muscle enhancer according to the present invention, the return to daily life and sports activities of a subject having muscle atrophy or muscle damage can be accelerated. Therefore, the present invention can be used in the treatment of muscle atrophy and muscle damage.

### [Sequence Listing Free Text]

SEQ ID NO: 1: Primer for sense strand of Trim63
SEQ ID NO: 2: Primer for antisense strand of Trim63
SEQ ID NO: 3: Primer for sense strand of Mstn
SEQ ID NO: 4: Primer for antisense strand of Mstn
SEQ ID NO: 5: Primer for sense strand of Gapdh
SEQ ID NO: 6: Primer for antisense strand of Gapdh

## Claims

1. A skeletal muscle enhancer comprising spermidine or a pharmaceutically acceptable salt thereof, which is administered intramuscularly.

2. The skeletal muscle enhancer according to claim 1, which is administered to a subject having skeletal muscle atrophy.

3. The skeletal muscle enhancer according to claim 1 or 2, wherein the skeletal muscle atrophy is sarcopenia or disuse muscle atrophy.

4. The skeletal muscle enhancer according to claim 1, wherein the skeletal muscle enhancer is administered to a subject having skeletal muscle damage.

5. The skeletal muscle enhancer according to claim 4, wherein the skeletal muscle damage is a muscle strain.

6. The skeletal muscle enhancer according to claim 4, wherein the skeletal muscle damage is due to myogenic disease.

7. The skeletal muscle enhancer according to any one of claims 1 to 6, wherein the skeletal muscle is enhanced by suppression of degradation or promotion of synthesis of skeletal muscle.

8. A therapeutic agent for skeletal muscle atrophy comprising spermidine or a pharmaceutically acceptable salt thereof, which is administered intramuscularly.

9. A therapeutic agent for skeletal muscle damage comprising spermidine or a pharmaceutically acceptable salt thereof, which is administered intramuscularly.

10. A skeletal muscle homeostasis restoring agent comprising spermidine or a pharmaceutically acceptable salt thereof, which is administered intramuscularly.
